(19) Europäisches Patentamt
European Patent Office
Office européen des brevets

(11) EP 4 616 794 A1

(12) EUROPEAN PATENT APPLICATION

(43) Date of publication:
17.09.2025 Bulletin 2025/38

(21) Application number: 24305365.9

(22) Date of filing: 11.03.2024

(51) International Patent Classification (IPC):
A61B 5/021 (2006.01)          A61B 5/00 (2006.01)

(52) Cooperative Patent Classification (CPC):
A61B 5/02141; A61B 5/6824

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Hinlab
92200 Neuilly-Sur-Seine (FR)

(72) Inventor: de BEGON de LAROUZIERE de
MONTLOSIER, Denys-Michel
75006 Paris (FR)

(74) Representative: Icosa
83 avenue Denfert-Rochereau
75014 Paris (FR)

(54) WEARABLE DEVICE FOR MEASURING BLOOD PRESSURE

(57)    The invention relates to a wearable device (100) for measuring blood pressure comprising a sealed housing (25) including a pump in fluidic connection with a bladder (15) configured to exert a pressure on the at least one portion of a limb of a subject, at least one vent (50) positioned on at least one wall of said sealed housing (25), the at least one vent (50) being configured to connect an inside of the sealed housing (25) with an outside environment to allow air to be pumped in the sealed housing (25). The invention is characterized in that the at least one vent (50) comprises at least one waterproofing element configured to allow air to transit between the inside of the sealed housing (25) and the outside environment while preventing water from entering the sealed housing (25).

Fig. 1

**Description**

**FIELD OF INVENTION**

The present invention pertains to the field of devices and methods for physiological parameter estimation. In particular, the invention relates to a wearable device for measuring blood pressure.

**BACKGROUND OF INVENTION**

Traditional wearable devices for measuring blood pressure comprise an inflatable cuff configured to be wrapped around a limb of a subject. The cuff may be inflated and deflated using a pump. As most electronic devices, the pump and the associated electronics are included and protected by a housing.

Several issues with traditional wearable devices for measuring blood pressure have arisen. Indeed, they are very frequently used during auscultation at a doctor's office or in a hospital. On average, a same device may be used, for instance, up to 20 times per days on different subjects. Between each subject, the device has to be cleaned and disinfected. The hygiene guidelines regarding devices for measuring blood pressure are to immerse completely or partially the device in a cleaning and disinfecting solution such as water and detergent, enzymatic cleaners, or hospital-grade cleaning agents such as glutaraldehyde, hydrogen peroxide, ortho-phthalaldehyde, and peracetic acid with hydrogen peroxide. Additionally, the device may be further cleaned using wipes.

However, when entirely immersed in a disinfecting solution several times a day, traditional blood pressure measuring devices may see their electronics and pump be damaged. It is therefore essential to house the electronics inside a sealed housing to prevent such damage.

However, is important to understand that pressure may vary inside the sealed housing. For example, the pressure inside the sealed housing may increase when sunlight hits the device or when the temperature rises. Conversely, as the temperature drops, so does the pressure.

This pressure must escape from the device's sealed housing, otherwise the device's seals are subjected to constant pressure that may ultimately compromise the sealed housing and induce damage to the electronics and pump.

Moreover, if the entire housing of the device is sealed, the question arises of the air drawn in by the pump, as the pump is originally located inside the sealed housing.

There is therefore a need for a solution allowing the air to penetrate into the sealed housing, while maintaining the watertightness required for IPX7 standards.

The problem which the invention thus seeks to solve is to develop a wearable device for measuring blood pressure that is waterproof, while still being capable of allowing air to enter into the housing to inflate and deflate the bladder comprised in the cuff.

**SUMMARY**

This invention thus relates to a wearable device for measuring blood pressure of a subject comprising:

- a control unit comprising a sealed housing, said sealed housing comprising a pump;
- a cuff configured to wrap around at least one portion of a limb of said subject, the cuff comprising a bladder configured to exert a pressure on the at least one portion of a limb of said subject, said bladder being in fluidic connection with said pump so as to be inflated;
- at least one vent positioned on at least one wall of said sealed housing, the at least one vent being configured to connect an inside of the sealed housing with an outside environment to allow air to be pumped in the sealed housing by the pump to inflate the bladder, characterized in that the at least one vent comprises at least one waterproofing element configured to allow air to transit between the inside of the sealed housing and the outside environment while preventing water from entering the sealed housing.

According to the invention, the outside environment typically refers to the surrounding atmosphere or the space external to the device itself. The pump is drawing in air from the outside environment to generate the necessary pressure or vacuum to inflate or deflate the bladder.

According to the invention, a vent is a structure or device designed to provide a means for the passage of gases, such as air, or other substances, between the interior and exterior of an enclosure or space. The vents from the invention also allow to maintain proper air circulation, control temperature, release excess pressure, or prevent the buildup of harmful gases within the sealed housing. For instance, when the wearable device of the invention is used for prolonged period of time, the electronic components release heat in the sealed housing, which has an effect of building up the pressure inside the sealed housing. The vents are configured to relieve such pressure to preserve the integrity of the sealed housing.

The at least one waterproofing element comprised in the at least one vent provides a way to obtain a wearable device for measuring blood pressure that is waterproof, while still maintaining the above-mentioned functionalities of the vents. Especially, it enables to offer a blood pressure measuring device that can resist frequent uses and a difficult environment, and especially the hospital environment.

The system according to the invention may comprises one or several of the following features, taken separately from each other or combined with each other.

According to one embodiment, the at least one vent comprising the at least one waterproofing element is configured to allow air to transit with an air flow of at least 1 L/min.

This minimum airflow is the one required for the pump to inflate the bladder in such a way that it is possible to measure the blood pressure of the subject (e.g. with enough pressure to cut-off blood circulation).

According to a first embodiment, the at least one waterproofing element comprises a labyrinthine conduit that connects the inside of the sealed housing with the outside environment while preventing water from entering the sealed housing.

According to the invention, a labyrinthine conduit refers to a complex and convoluted pathway designed to impede the flow of water. In the context of waterproofing, a labyrinthine conduit consists of a series of channels, chambers, or passages arranged in a complex manner. These pathways are engineered to create multiple barriers and obstructions that water must navigate through, thereby slowing down its progress and reducing the likelihood of water ingress into the device. The labyrinthine conduit may be constructed using various materials and methods, such as creating baffles or barriers within the conduit, or utilizing specialized designs to maximize the tortuosity of the pathway. By forcing water to navigate through this intricate network, the labyrinthine conduit enhances the waterproofing capabilities of the device, providing a more effective barrier against water intrusion.

More precisely, according to one embodiment, said at least one vent comprises an entry leading to the outside environment and an exit leading to the inside of the sealed housing and a pathway connects said entry to said exit, said pathway comprising at least one bend.

According to the invention, a "bent" refers to a curved or angled section within the pathway of the labyrinthine conduit.

Preferably, the pathway comprises at least two bents, wherein each bent forms an angle of at least 90 degrees.

According to another embodiment, the at least one waterproofing element comprises at least one membrane.

A membrane is a selectively permeable barrier or structure composed of thin layers of molecules or materials that separate two regions or compartments, allowing for controlled passage of certain substances while restricting or blocking the passage of others based on factors such as size, charge, or chemical properties. According to the invention, the membrane is configured to selectively allow air molecules (e.g. hydrogen, oxygen, nitrogen) while restricting or blocking the passage of water molecules.

According to one embodiment, the membrane comprises pores with a diameter comprised between 0.02 $\mu$m and 0.5$\mu$m.

The diameter of the pores allows air molecules (including dioxygen and nitrogen but excluding water vapor), that have a much smaller diameter of about 0.3 nm to pass through the membrane pores, while water droplets, that have a diameter comprised between 20 $\mu$m and 100 $\mu$m and also impurities cannot pass through the pores.

According to one embodiment, the pores have a density ranging from 70% to 90% and preferably of 82%.

According to the invention, the density refers to the fraction of the membrane's total area or volume that is occupied by pores. This percentage is calculated by dividing the area or volume of the pores by the total area or volume of the membrane and then multiplying by 100 to express the result as a percentage. Such density may be measured using methods such as Mercury Intrusion Porosimetry, Gas Adsorption (for example using the Brunauer-Emmett-Teller (BET) method), Scanning Electron Microscopy (SEM) or Atomic Force Microscopy (AFM).

According to one embodiment, the membrane has a resistance of a minimum of 10 kPa, and preferably of a minimum of 30 kPa and more preferably of at least 50 kPa.

The specified resistance corresponds to a water resistance of the membrane, as measured according to the Japanese Industrial Standards (JIS) L 1092 high water pressure method.

According to one embodiment, the membrane is made of a hydrophobic material configured to repel water vapor molecules.

According to one embodiment, the membrane is made of polytétrafluoroéthylène (PTFE).

The membrane may be integrated to the vent in several ways.

According to one embodiment, the membrane comprises an adhesive structure configured to be adhered on a portion of said at least one wall of said sealed housing wherein said at least one vent is positioned, said portion surrounding said at least one vent.

The waterproofing element may also comprise at least one membrane and at least one labyrinthine conduit.

**DEFINITIONS**

In the present invention, the following terms have the following meanings:

"**IPX7 standard**" refers to is a rating used to classify the level of waterproofing or water resistance in electronic devices and other equipment. "IP" stands for "Ingress Protection," and "X" may be replaced by a number indicating the degree of protection against solids such as dust and against liquids such as water. The "7" indicates the level of water resistance. The device is tested so that it should withstand immersion in water up to 1 meter for up to 30 minutes without water seeping into its internal components.

"**The Japanese Industrial Standards (JIS) L 1092 high water pressure method**" is a testing standard used to evaluate the waterproofness or water resistance of fabrics and materials. This method is commonly employed in the textile industry, particularly for products such as rainwear, outdoor gear, and waterproof garments. In the JIS L 1092 high water pressure method, a specific area of the material to be tested is subjected to a continuous stream of water at a predetermined pressure for a specified duration. The pressure applied typically ranges from 0.05 MPa (megapascals) to 0.2 MPa. The duration of the test may vary but is commonly set at a minimum of a few minutes. In the JIS L 1092 high water pressure method, a specific area of the material to be tested is subjected to a continuous stream of water at a predetermined pressure for a specified duration. The pressure applied typically ranges from 0.05 MPa (megapascals) to 0.2 MPa, which is equivalent to approximately 0.5 kgf/cm$^2$ to 2.0 kgf/cm$^2$. The duration of the test may vary but is commonly set at a minimum of a few minutes.

## BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration, purely illustrative and non-limiting examples, with reference to the accompanying drawings:

- **figure 1** is a perspective view of an embodiment of the device according to the present invention,
- **figure 2** is a perspective view of an open control unit according the present invention,
- **figure 3** is a schematic illustration of the fluid circulation between the inside and the outside of the device according to the present invention,
- **figures 4a, 4b, 4c** are side views from different embodiments of a sealed housing according to the present invention,
- **figures 5a, 5b** are schematic examples of a vent presenting a labyrinthine conduit according to a possible embodiment of the present invention,
- **figures 6a, 6b** and **6c** are schematic examples of three different embodiments of a vent presenting a membrane according to the present invention, and
- **figures 7a, 7b** are schematic examples of labyrinthine conduits according to two possible embodiments of the present invention.

## DETAILED DESCRIPTION

The present invention is about a wearable device **100** for measuring the vital signs and more particularly the blood pressure of a subject. The wearable device **100** presents a general circular shape in order to easily fit or wrap around a limb, preferably an arm of a subject.

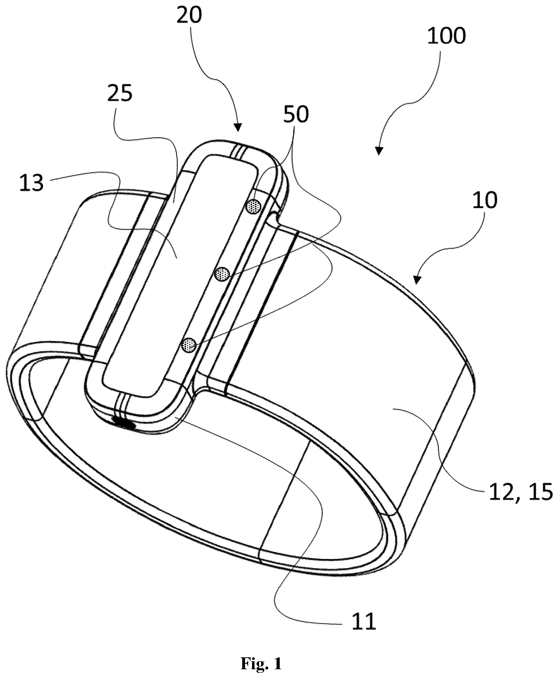

As illustrated on Figure 1, the wearable device **100** for measuring blood pressure comprises:

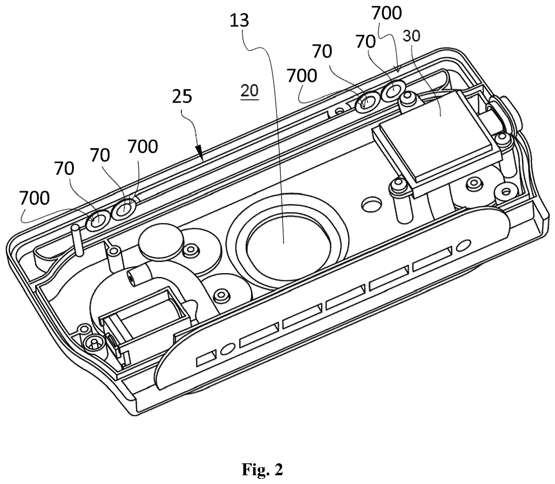

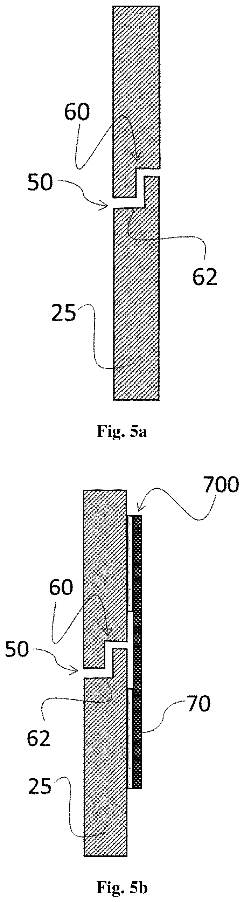

- a control unit **20** comprising a sealed housing **25**, said sealed housing **25** comprising a pump **30** (see Figure 2),
- a cuff **10** configured to wrap around at least one portion of a limb of said subject, the cuff **10** comprising a bladder **15** configured to be inflated by the pump **30** in order to exert a pressure on the at least one portion of a limb of said subject,
- at least one vent **50** positioned on at least one wall of the sealed housing **25**, comprising at least one waterproofing element **60, 70** (see Figure 5a to Figure 6c).

The control unit **20** (see Figure 2) comprises all the electronics and pumping means (including the pump **30**) gathered and protected inside the sealed housing **25**.

The control unit **20** comprises a sealed housing **25** with a substantially parallelepiped shape. Preferably, the sealed housing **25** has a length ranging from 5 cm to 10 cm, a width ranging from 2 cm to 5 cm and a thickness ranging from 0.5 cm to 3cm. Corners of the sealed housing **25** may have a more rounded shape for aesthetic purpose. The sealed housing **25** may be made of any kind of solid or flexible material, such as: polypropylene, ABS, PVC, Polyamide, rubber, resin, silicone, urethane, nylon, glass such as Gorilla®, or titanium. Such material is also chosen to be waterproof.

The control unit **20** may be opened by means of a removable lid, not represented in the figures. The removable lid may be the upper or lower wall of the sealed housing **25**.

The control unit **20** may either be removable from the cuff **10** or be permanently attached to the cuff **10** (see description further below).

The control unit **20** may also comprise a power supply device, not represented in the figures, configured to supply power to the components of the control unit **20**. Said power supply device may comprise a built-in battery and/or removable battery. In one embodiment, the removable battery is rechargeable. In another embodiment, the built-in battery has capacity comprised between 10 mAh and 300 mAh, preferably between 30 mAh and 100 mAh. This embodiment ensures that the built-in battery duration is long enough to ensure the continuity of the functions whilst the removable battery is not in place, for instance while the removable battery is being replaced or recharged. For instance, the continuity of the functions may be ensured for at least 30 minutes; preferably for at least 40 minutes, 50 minutes or 60 minutes.

As the wearable device **100** for measuring blood pressure may be configured to work for the auscultatory technique or the oscillometric technique, the control unit **20** comprises different elements, depending on the chosen technique.

The first technique is based on the Korotkoff sounds of the subject recorded during the cuff **10** inflations and deflations. The medical staff may parameter the control unit **20** to inflate and deflate the bladder **15** while listening to the Korotkoff sounds with a stethoscope. Alternatively, the wearable device **100** for measuring blood pressure may include a sensor configured to automatically record the Korotkoff sounds. Such sensor may be a PPG sensor for instance.

The second technique estimates the systolic and diastolic pressures of the subject based on the amplitude of the pressure oscillations recorded within the cuff **10** during deflation or inflation. In that case, the control unit **20** includes at least one pressure sensor. This allows to monitor the pressure in the bladder **15** and to obtain the blood pressure of the subject therefrom, via the oscillometric technique or "cuff-based" technique.

More precisely, oscillometry is a technology based on the principle of occluding blood flow within the subject's limb with the inflatable cuff. Unlike the auscultation method, it measures the pulses transduced through the inflatable cuff, and exploits the fact that the pulse amplitude is modulated by the difference between the mean arterial pressure and the applied pressure in the cuff. The pulses amplitude increases when the applied pressure is between the subject's diastolic blood pressure and the mean arterial pressure, while the pulses amplitude decreases when the applied pressure is between the mean arterial pressure and the systolic blood pressure. Variation of pulses amplitude with the applied pressure yields a quasi-symmetric waveform from which the systolic and diastolic blood pressures can be determined using empirical formulas. Advantageously, the oscillometric technique allows to independently measure systolic and diastolic blood pressures. Moreover, the oscillometric technique provides an absolute blood pressure value.

The pressure sensor may be of any type, for instance it may be a piezoresistive pressure sensor.

In one embodiment, the wearable device **100** measures pressure during a controlled deflation of the bladder **15**. Preferably, in another embodiment, the wearable device **100** measures blood pressure during the inflation of the bladder **15**. This method ensures the subject's comfort because of the shorter occlusion time of their artery due to the pression exerted from the cuff **15** on the subject's limb. It also has a reduced battery consumption.

In some other embodiment(s), the blood pressure value may further be obtained using several techniques at the same time. For instance, a first blood pressure value may be obtained using a cuff-based technique and a second blood pressure value may be obtained using an optical sensor. Therefore, the control unit **20** may also comprise at least one optical sensor. The optical sensor is arranged so that it is in close contact with the subject's skin to allows to acquire a signal at an elevated acquisition frequency, which is suitable for a continuous or regular longitudinal monitoring of the subject's blood pressure. Preferably, the optical sensor is a photoplethysmographic (PPG) sensor comprising a light source and a light detector, more preferably a single-wavelength PPG sensor. For instance, the light source may be an infrared LED. In one embodiment, the light source emits an infrared radiation from about 700 nm to about 1000 nm, preferably from about 900 nm to about 980 nm. In one preferred embodiment, the light source is configured to emit an infrared radiation of about 940 nm. The infrared radiation wavelengths herein mentioned preferably are the centroid wavelengths or the peak wavelengths of the light emitters at their operating temperature. The wearable device **100** may comprise several optical sensors, which may be structurally independent or embedded within a sensor array. In one particular example, the wearable device **100** comprises at least three optical sensors and preferably: at least one infrared LED, at least one green LED, at least one red LED. Preferably, said infrared LED, green LED and red LED are configured to emit a radiation having a radiation wavelength of 940 nm, 536 nm, and 655nm, respectively. Advantageously, the optical sensor may further comprise a light filtering element configured to minimize the intensity of the light reflected from the skin surface without affecting the light reflected by subcutaneous tissues. The light filter element may comprise a first sheet of polarized glass placed on the light source of the optical sensor and/or a second sheet of polarized glass placed on the light detector of the optical sensor.

In that embodiment, the sealed housing **25** may include an opening in its inferior wall configured to allow insertion of the optical sensor to take its measurements. To ensure waterproofness, the opening for the optical sensor may be protected by a cover glass, made either of transparent plastic or glass such as Gorilla glass®. The cover glass may be secured to the housing **25**, using waterproofing sealing techniques, including adhesives, gaskets, or other sealing materials. The opening is preferably made with a circular shape with a diameter for example comprised between 0.5 cm and 3 cm. In another embodiment, the sealed housing **25** may comprise a transparent material in front of the optical sensor to allow said optical sensor to make its measurements. The transparent material may be added to the sealed housing **25** through methods such as molding the plastic to form a transparent window or incorporating a separate transparent material within the sealed housing **25**.

Additionally, the control unit **20** may include other sensors such as a temperature sensor, a heat flux sensor, a position sensor, an accelerometer, a pressure transducer, an ultrasound sensor, an accelerometer, a gyroscopic sensor, an electrical sensor, such as for instance an electrical sensor comprising electrodes, a tomographic sensor, an acoustic sensor, a magnetometer, a humidity sensor, or even an impedance sensor. They may be used to calculate parameters such as the oxygen saturation, the heart rate, the respiratory rate, the blood pressure trend, the heart rate variability, the cardiac output, the skin temperature, the body temperature, or even the body composition.

The pumping means comprise the pump **30**, are part of the control unit **20** and are represented on Figure 2. The pump **30** may be a rolling air pump, a piezo electric pump, a resonant gas pump, a diaphragm/membrane gas pump, and the like.

The pump **30** is in charge of putting fluid in motion. Preferably, said fluid is air. The pump **30** is therefore in fluidic connection with the bladder **15** of the cuff **10**. The fluid may flow in a first direction, from the bladder **15** toward the pump **30** and in a second direction, from the pump **30** toward the bladder **15**.

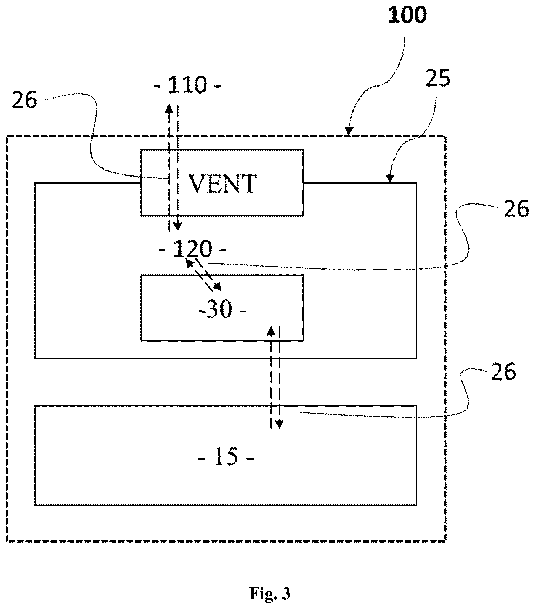

As schematically illustrated on Figure 3, the pump **30** is connected to the bladder **15** of the cuff **10** with a network of fluidic connections **26**. Said fluidic connections **26** may be flexible or rigid. The network of fluidic connections **26** may be built in one piece, for instance by molding or 3D printing. The resulting piece(s) may be fixed to the sealed housing **25**.

The network of fluidic connections **26** is further configured to connect the sealed housing **25** and the bladder **15** in an air tight and waterproof manner. The network of fluidic connections **26** preferably comprises first connection elements which allow fluid to enter the sealed housing **25** and second connection elements which are configured to let fluid exit the sealed housing **25**. The pump **30** is connected to both the first connection elements and second connection elements with a dedicated arrangement of tubing connections (not represented). The pump **30** is therefore configured to pump the fluid outside of the sealed housing **25** through the first connection elements and to pump the fluid inside the sealed housing **25**, through the second connection elements. Alternatively, in some other embodiments, there is only one connection element, through which the pump **30** may pump-in and out the fluid.

Both the first connection elements and the second connection elements may be positioned on the same side of the sealed housing **25** or alternatively, the first connection elements may be positioned on a first lateral side of the sealed housing **25** and the second connection elements may be positioned on the opposite lateral side of the sealed housing **25**.

At least one valve, such as a solenoid valve, not represented in the figures, may also be included inside the sealed housing **25**, in the pumping means, to control the passage of fluid through the network of tubing connections **29**, for instance by opening, closing, or partially obstructing at least one of the tubing connections **29**. Alternatively, a protection system could be included with a second valve in case of failure of the first valve, to ensure that there is no long-term arm compression of the subject. The valve may be physically separated from the pump **30** or integrated in the pump **30**. For instance, the pump **30** may comprise one or more blowers, which allows to discharge air, thereby providing a passive valve function.

The valve may be made waterproof using seals and gaskets around the valve, cable glands or similar devices to establish a waterproof seal around cables entering the valve, or by applying a protective coating to the valve's internal components, to safeguard against moisture. Potting may also be used on critical electronic components of the valve to prevent water from reaching sensitive area.

In another embodiment, the first connection elements may be connected to the pump **30** and the second connection elements may be connected to the valve.

In the embodiments of the wearable device **100** including a pressure sensor , said pressure sensor is connected to at least one of the first connection elements, while the pump **30** is connected to the second connection means. This configuration allows to get better pressure measurements in particular when the pump **30** is activated during the blood pressure measurement. In another embodiment, the pump **30** and the pressure sensor are connected to the same connection element. Advantageously, a protection system could be included with a second pressure sensor in case of failure of the first pressure sensor, to ensure that the subject's limb is not compressed for an extended period of time.

To ensure waterproofness of the sealed housing **25**, adhesives and sealants, may be used to create a strong and durable bond between the different parts of the sealed housing **25**, such as Loctite® adhesives, thus sealing potential entry points for water. Alternatively, silicone sealant may be used for the seams and openings in the sealed housing **25** to create a water-resistant barrier. Ultrasonic welding may also be used to fuse plastic components of the sealed housing **25** together using ultrasonic vibrations, thus creating a watertight seal.

Moreover, rubber gaskets and/or O-rings at joints and seams in the sealed housing **25** may help creating a tight seal, preventing water from infiltrating the electronic components. Additionally, a layer of epoxy resin, hydrophobic coating or nano coating may be applied to the sealed housing **25** to provide a waterproof barrier, protecting the internal electronics from water exposure. In the same manner, the electronic components may also be coated with a protective coating to protect them from moisture, dust, and other contaminants. In other embodiments, the sealed housing **25** may be filled with potting compounds such as urethane, to help encapsulate and protect the electronic components from water and environmental damage.

The cuff **10** is configured to be worn around a portion of a limb of the subject, preferably around an arm of the subject, and more particularly around their upper arm.

When worn around the upper arm, the wearable device **100** is less sensitive to motion artifacts compared to wearable devices worn around the wrist or a finger. Moreover, the upper arm is characterized by elevated vascularization, and presence of main arteries such as the brachial artery. Therefore, in those embodiments, the blood pressure measured with the present wearable device **100** is indicative of the blood pressure in the arm arteries, such as the brachial or humeral artery, which is close to the blood pressure in the aorta. This allows to provide a highly accurate and non-invasive blood pressure estimation.

The cuff **10** may either be directly connected to the sides of the sealed housing **25** or alternatively, the cuff **10** may comprise a docking station **11** (see Figure 1) configured to receive and removably secure the control unit **20**. Said docking station **11** will be described in details further below.

The cuff **10** has preferably a length for example ranging from 20cm to 42 cm, preferably from 20 cm to 36 cm or from 20 cm to 30 cm. The bracelet **12** has a width that may range from 5 cm to 16 cm, preferably from 9 cm to 14 cm.

An inner surface and an outer surface of the cuff **10** may be defined, the inner surface being configured to be in contact with the skin of the subject when the device **100** is worn. A first extremity of the cuff **10** may be permanently connected to the docking station **11** or the control unit **20** while the second extremity can be used to adjust the size of the cuff **10** to the limb portion size. The second extremity **102** may thus be removably connected to the control unit **20** or the docking station **11**, thus allowing to open the cuff **10** to put on or remove the wearable device **100**. In an alternative embodiment, the second extremity of the cuff **10** is also permanently connected to the docking station **11** or the control unit **20** and the cuff **10** comprises an adjustment system presenting an elastic segment. The wearable device **100** can thus be put on or removed simply by deforming the elastic portion.

The bladder **15** may be directly connected to the cuff **10** or be part of a bracelet **12** configured to be connected and secured to the cuff **10**. In some embodiments, the bracelet **12** could be removably secured to the cuff **10**.

The bracelet **12** or cuff **10** may be obtained using two bands of fabric sawn together to form a sleeve where the bladder **15** can be fitted. The bracelet fabric may be a nylon or synthetic fiber fabric, which protects the bladder **15** from cuts during use by on-scene rescue technicians and reduces subject discomfort. The bracelet fabric is preferably very flexible to adapt to the bladder **15** inflations and deflations, durable and waterproof to resist numerous uses. Alternatively, the bladder **15** may be directly exposed and not covered with a protective sleeve. In that case, the bladder **15** is preferably made of a thicker material to withstand wear.

The bladder **15** is inflatable to a pressure that is high enough to uniformly restrict the blood flow in the subject's arm around which the bracelet **10** is worn. In other words, the bladder **15** may be inflatable up to a maximum inflation pressure which is superior to the pressure needed to occlude the brachial artery. Said maximum inflation pressure may be equal to 250 mmHg, preferably 260 mmHg, 270 mmHg or 280 mmHg. In some cases, it may be desirable to provide a bladder **15** inflatable up to a pressure of 300 mmHg.

The bladder **15** may be made of a single inflatable chamber that does not extend throughout the length of the cuff **10**. For instance, the bladder **15** may extend for a length ranging from 20cm to 42cm preferably from 22cm to 36cm., so as to cover for instance 80% of the wearer's arm. Alternatively, the bladder **15** may extend throughout the length of the cuff **10**. Alternatively, the bladder **15** may contain several inflatable chambers distributed along the cuff **10**. The bladder **15** may be made of resin, rubber, latex, silicon, neoprene, polyester, synthetic fibers, Polyurethan (PU), Thermoplastic Polyurethane (TPU), polyvinyl chloride (PVC), Polyamide (PA), Vinyl, Nylon, Polyester or a combination thereof. Additionally, the material may be coated with a water-resistant material.

In particular, TPU is a versatile material known for its durability, flexibility, and resistance to abrasion and chemicals. One advantage of the TPU is that it can be directly a single-cuff that merge the bladder and the cuff around.

The cuff **10** only partially circles the limb portion. The remaining portion is completed by the presence of the control unit **20** or the docking station **11**.

The bladder **15** includes an opening in which a tubing connection may be fitted, in order to connect the bladder to the pump **30** of the device **100**.

In case the cuff **10** is not permanently secured to the sealed housing **25** of the control unit **20**, the control unit **20** may be plugged and unplugged on the cuff **10** at will via a docking station **11**.

To that end, the control unit **20** may be fitted inside the reception site of the docking station **11** in several ways. In order to do so, the docking station **11** comprises a bottom wall configured to come in contact with the skin of the subject, framed by a first lateral wall and a second lateral wall, forming together a reception site configured to receive the control unit **20**. For instance, the reception site may have a complementary shape to the control unit **20**. The reception site may thus have a parallelepiped shape with a length for example ranging from 5 cm to 10 cm, a width for example ranging from 2cm to 5cm and a thickness for example ranging from 0.5 cm to 3 cm.

The control unit **20** may be inserted by a movement from top down. Alternatively, the control unit **20** may be inserted by a sliding movement from one side to the other. In that case, the control unit **20** may include a rail cooperating with a slide on the docking station side walls. Once installed on the docking station **11**, the control unit **20** may either extend over the ends of the docking station **11** or be completely enclosed inside the reception so that it is protected from chocs and cannot be easily dislodged.

The control unit **20** may be maintained in the reception site using fixing means, such as deformable ergots, or a plug and socket system. Alternatively, the fixing means is a system of clip with male ergots on the reception site and female ergots on the control unit **20.** Moreover, the control unit **20** may present slots to receive such fixing means.

If necessary, the docking station **11** may comprise a see-through hole in its bottom wall. The see-through hole is configured to allow the optical sensor of the control unit **20** to perform its measurement on the skin of the subject. Preferably, the see-through hole is made with a circular shape with a diameter comprised between 0.5 cm and 3 cm. The see-through hole in the docking station bottom wall is configured to be aligned with the opening of the control unit **20** to allow the optical sensor to protrude through the see-through hole and come in close contact with the subject's skin.

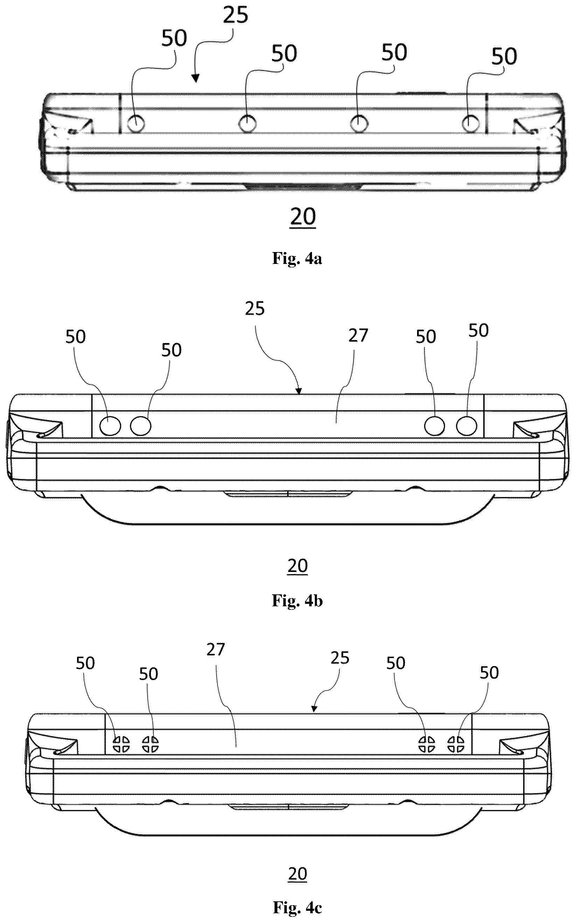

The device **100**, and more particularly the sealed housing **25** of the control unit **20** comprises at least one vent **50**. For instance, the device **100** may comprise one vent **50**, two vents **50**, three vents **50** and up to ten vents **50**, while preferably comprising four vents **50.** In some embodiments, the vents **50** may be positioned anywhere on the sealed housing **25.** For instance, the vents **50** may be regularly distributed along at least one side wall **27** of the sealed housing **25** (see figure 4a). The vents **50** may for instance be positioned on a same side wall **27** or distributed on different side walls. The vents **50** may be spaced from each other by a distance of at least 1 mm. The maximum distance between the vents **50** is conditioned by the length of the device **100.**

In an alternative embodiment, the vents **50** are located at both extremities of a side wall **27** of the sealed housing **25.** For instance, two vents **50** may be positioned on one extremity and two other vents **50** may be positioned on the other extremity, the two pairs of vents **50** being spaced from each other by a distance comprised between 1 mm and 10 cm (see Figure 4b and Figure 4c).

Each vent **50** of the device **100** is configured to connect the inside **120** of the sealed housing **25** with the outside environment **110** of the device **100** (see Figure 3). As a consequence, the vent **50** may have a longitudinal length (e.g. corresponding to the thickness of the housing wall) comprised between 1 mm and 5 cm.

Vents **50** are essential to the operation of high-end technical electronic devices. They allow pressure to escape, while protecting the electronics from external contaminants that could enter the sealed housing **25.** They also facilitate cooling and help prevent condensation. All these features contribute to the wearable device **100** smooth operation and long service life.

More particularly, as illustrated on Figure 3, each vent **50** from the present invention allows:

- to maintain proper air circulation inside the sealed housing **25**, more particularly to allow air to be pumped inside the sealed housing **25** by the pump **30** in order to inflate the bladder **15**,

- to control the inside temperature of the sealed housing **25** (and thus of the control unit **20**),
- to release excess pressure from the inside **120** of the sealed housing **25** toward the outside environment **110**,
- to prevent the buildup of harmful gases inside the sealed housing **25.**

Roughly formulated, each vent **50** is a hole inside the sealed housing **25.** Each vent **50** comprises an entry leading to the outside environment **110** of the device **100** and an exit leading to the inside **120** of the sealed housing **25.** The entry and the exit are connected by a pathway. In the present invention, each vent **50** is configured to enable a specific air flow. It can be through one big vent **50**, or several smaller vents **50.** An advantage of several small vents **50** is that it increases the resistance of each single vent **50** (smaller area) and also, it is less visible.

The diameter of each vent **50** may be comprised between 1 mm and 29 mm, and preferably between 1 mm and 10 mm, typically equal to 7.6 mm each diameter being associated with a specific air flow capacity. The vents **50** are configured to enable an air flow of at least 1 L/min and for example up to 5 L/min. All the vents **50** may either present different diameter or preferably the same diameter.

More precisely, the total flow rate into the sealed housing **25** is the sum of the flow rates through each vent **50.** In the example depicting four vents **50**, each vent **50** may allow air to pass through at a flow rate ($Q_{vent}$) of 282 mL/min. The total flow rate into the housing **25** is the sum of the flow rates through each vent **50.** In this case, the total flow rate ($Q_{total}$) is equal to 4*282 mL/min = 1128 mL/min.

The relationship between the surface area of a circular vent, such as a membrane with a specific diameter, and the airflow through it is described by the following equation, derived from principles of fluid dynamics and commonly used to model the volumetric flow rate through an orifice:

$$Q = A \times C_d \times \sqrt{\left(\frac{2 \times \Delta P}{\rho}\right)}$$

in which:

- Q is the volumetric flow rate, representing the amount of fluid (in this case, air) passing through the vent per unit of time,
- A is the effective orifice area, which accounts for the geometric characteristics of the vent. For a circular vent, such as a membrane with diameter D, the effective orifice area (A) is calculated as A = ($\pi$ *

D^2) / 4,

- Cd is the discharge coefficient, a dimensionless factor that reflects the efficiency of the vent design. It considers factors such as turbulence, boundary layer effects, and other non-idealities,
- $\Delta P$ is the pressure drop across the vent. It represents the difference in pressure between the upstream and downstream sides of the vent,
- $\rho$ is the density of the fluid, in this context, air.

The discharge coefficient ($C_d$) is a critical parameter in the correlation between surface area and airflow. Its determination involves experimental measurements and depends on the specific vent geometry, surrounding conditions, and the characteristics of the fluid. For instance, the discharge coefficient ($C_d$) may be equal to 10.

As an example, it is considered a circular membrane with a diameter of 7.6 mm. The effective orifice area (A) for this membrane is calculated as A = $(\pi*7.6mm)^2/4$ = 14.44 mm$^2$. Therefore, the total effective office area for four vents is equal to approximately 57.76 mm$^2$. The pressure drop is equal to $Q_{total}/(A_{total}*C_d)^2*1/2$ = 1128/(57.76* 10)$^2$* 1/2 = 1.190 mmHg.

It has to be noted that the calculated values are based on theoretical calculations and have not been empirically validated. These results serve as an illustrative example to provide an order of magnitude for the physical values at play.

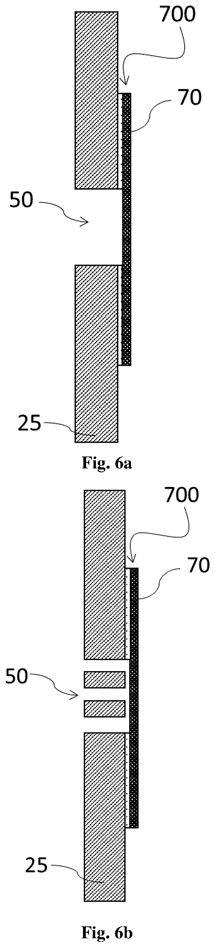

In case of a larger vent **50**, another embodiment, like the one depicted on Figure 6b would be to have a larger vent global opening, comprising several smaller holes inside the sealed housing **25** or, to put it in another way, the vent **50** may comprise strengthening means that cross through the opening, thus reducing the overall opening surface. For instance, in some embodiments, the vents **50** may present a strengthening mean in the shape of a cross, to make it more rigid (see figure 4c).

In order to avoid any water to be transported inside the sealed housing **25** of the control unit **20**, each vent **50** comprises at least one waterproofing element **60, 70**. Each waterproofing element **60, 70** is thus configured to allow air to transit between the inside **120** of the sealed housing **25** and the outside environment **110** of the device **100** while preventing water from entering the sealed housing **25**.

In one embodiment present on Figure 5a and Figure 5b, at least one or each of the waterproofing element(s) **60, 70** comprise(s) a labyrinthine conduit **60** that connects the inside **120** of the sealed housing **25** with the outside environment **110** of the device **100** while preventing water from entering the sealed housing **25**.

The labyrinthine conduit **60** may for example be designed inside a plastic plug configured to be secured inside the opening of the vent **50**. In those embodiments, this labyrinthine conduit **60** is preferably constructed using a waterproof and durable plastic material.

The pathway of the labyrinthine conduit **60** comprises at least one bend **62** and the labyrinthine conduit **60** is thus directly created inside the wall of the sealed housing **25** (see Figure 5a and Figure 5b). In practice, this labyrinthine conduit **60** involve the use of intricate paths and barriers to create an extended path that prevents water from entering the housing **25**. It serves as a barrier that compels water to follow a labyrinthine path, thus preventing direct contact with the sensitive electronics and components of the control unit **20**. More precisely, the labyrinthine conduit **60** may be designed in various configurations, such as a maze, to maximize the distance water must travel. The labyrinth design leverages the principles of gravity and capillary action to guide water away from the sensitive electronics of the control unit **20** inside the sealed housing **25**. Water thus enters the labyrinthine conduit **60** but is forced to follow a complex path, which slows its progress and redirects it away from the electronic components of the control unit **20**. The labyrinthic design thus ensures the safety and functionality of the control unit **20** even in wet or underwater environments.

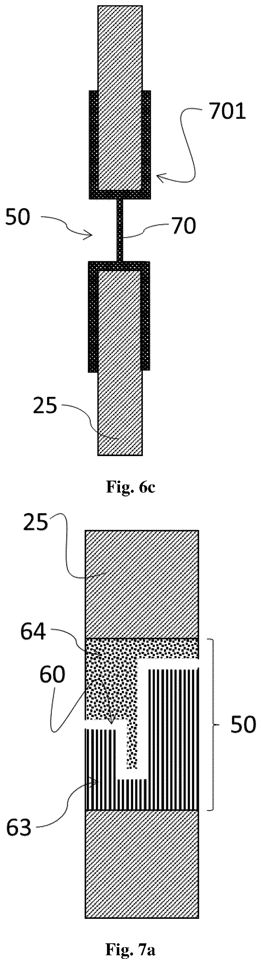

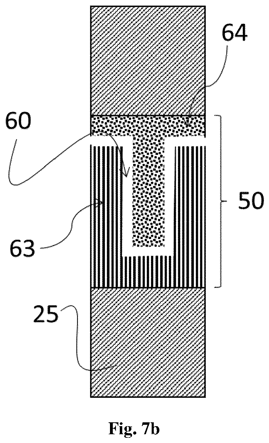

Such labyrinthine conduit **60** may be obtained using a first element **64** configured to cooperate with a second element **63** in such way that the interface between the first element **64** and the second element **63** forms the labyrinthine conduit **60** (see Figure 7a and Figure 7b). The first element **64** and the second element **63** may be inserted into the vent **50** opening and sealed to the walls of the vent **50** in a waterproof manner (e.g. using gaskets, or other sealing methods such as ultrasonic welding). The cooperation between the first element **64** and the second element **63** may be obtained using a groove (e.g. a recessed or channel-like indentation in the interface surface between the first element **64** and the second element **63**) formed on either one of the first element **64** and the second element **63** and a lip (e.g. a protruding edge or rim along the interface surface between the first element **64** and the second element **63**) formed on the other element.

Specifically, Figure 7a and Figure 7b show two examples of labyrinthine conduit **60**. In both Figures 7a and 7b, the labyrinthine conduit **60** comprises four bents **62**. However, in Figure 7b the path that water should travel to enter into the sealed housing 25 is much longer than in Figure 7a. The labyrinthine conduit **60** in Figure 7b is therefore more complex for water to cross. The bents **62** are generally formed by an angle in the pathway of the labyrinthine conduit **60**. The angle may be comprised between 1° and 360° an preferably comprised between 45° and 120°.

Additionally or alternatively (see Figure 5b, Figure 6a, Figure 6b and Figure 6c), each waterproofing element **60, 70** comprises at least one membrane **70**. For instance, the waterproofing element **60, 70** may comprise only one membrane **70** or a superposition of several membranes **70** with different characteristics. Moreover, the waterproofing element **60, 70** may comprise a combination of at least one membrane **70** and a labyrinthine conduit **60**.

The airflow through the membrane 70 may be comprised between 2000 mL/min/cm$^2$ and 4000 mL/min/cm$^2$, typically 3300 mL/min/cm$^2$.

In order to obtain a minimal flow rate of 1 L/min through the vent, the minimum diameter of the circular pathway (e.g. the diameter of the vent **50**) needed to achieve a flow rate of 1 L/min is approximately 0.62 cm.

Each membrane **70** is a waterproof air-permeable membrane. Preferably, each the membrane **70** comprises pores with an average diameter comprised between 0.02 $\mu$m and 0.5$\mu$m and preferably from 0.2 $\mu$m to 0.3 $\mu$m, so that water droplets that have a diameter comprised between 20 $\mu$m and 100 $\mu$m and impurities cannot pass through the pores and air molecules (including dioxygen and nitrogen but excluding water vapor), that have a much smaller diameter of about 0.3 nm can pass through. The pores may present a density (e.g. the proportion of the membrane's surface area that is occupied by pores. It is calculated by dividing the total area occupied by the pores by the total surface area of the membrane and then multiplying the result by 100 to express it as a percentage) ranging from 70% to 90% and preferably of 82%.

Each membrane **70** may have a thickness comprised between 20 $\mu$m and 500 $\mu$m, typically 260 $\mu$m.

Each membrane **70** further presents a resistance of at least 30 kPa, and preferably of at least 50 kPa, measured according to the Japanese Industrial Standards (JIS) L 1092 high water pressure method. This range underscores each membrane's ability to withstand water penetration effectively. This emphasis on water resistance aligns with the membrane's role in providing protection against external water sources, ensuring the subject and/or the underlying structure(s) to remain dry in adverse conditions. According to said standard:

- 10 kPa to 20 kPa: Materials with lower water resistance might be suitable for light rain or brief exposure to water,
- 20 kPa to 40 kPa: Medium-level water resistance is often appropriate for outdoor clothing, where the material needs to withstand moderate rainfall,
- 40 kPa to 70 kPa: High water resistance is typically required for more extreme conditions, such as heavy rain or prolonged exposure to water,
- 70 kPa and above: Materials with very high-water resistance are suitable for applications where the product needs to withstand heavy and sustained water pressure.

Each membrane **70** may be made of a material that is hydrophobic (e.g. a material that repels water, preventing its infiltration. When water comes into contact with the hydrophobic material, it forms distinct droplets due to the material's resistance to wetting). Indeed, water vapor molecules are much smaller than water droplets. The diameter of water vapor molecules can range from 0.0003 $\mu$m to 0.0004 $\mu$m. Due to the very small size of these water vapor molecules, they can pass through pores of average diameter comprised between 0.2 $\mu$m and 0.5$\mu$m. As a consequence, using a material that is hydrophobic allows a membrane **70** to repel water vapor molecules even when the pores have a diameter larger than the size of the water vapor molecules. This property arises from the molecular structure of the membrane material, which often includes nonpolar or low-polarity components, while water vapor molecules are polar. In that context, the non-polarity or low-polarity of the membrane repels polar water vapor molecules. Moreover, when water contacts the hydrophobic surface of the membrane **70**, it forms high-contact-angle droplets. This phenomenon arises because the cohesive forces holding water molecules together (surface tension) are stronger than the adhesive forces between water and the hydrophobic surface. High contact angles signify strong water repellence, causing water droplets to bead up and roll off the surface, incapable of infiltrating the material. The combination of hydrophobicity and small pore size in membranes **70** effectively prevents capillary action (e.g. the process where liquids are drawn into small spaces or pores due to adhesive forces). The material resists this action, ensuring that liquid water is unable to penetrate its structure.

Preferably, each membrane **70** is made of polytetrafluoroethylene (microporous PTFE). Microporous PTFE or Polytetrafluoroethylene, is a material that has an intricate network of minuscule pores distributed throughout its structure. These microscopic pores serve as the cornerstone of the material's exceptional properties. Here below, will be explained in detail why microporous PTFE permits the passage of air while effectively blocking water.

Moreover, microporous PTFE showcases remarkable resistance to chemical exposure, making it resilient against a wide range of acids, bases, and solvents. This chemical inertness ensures that its hydrophobic properties and pore structure remain intact even in the presence of various substances. Microporous PTFE also boasts an impressive temperature tolerance, withstanding extreme cold as low as -450°F (-268°C) and high heat up to 500°F (260°C). This wide temperature range further enhances its suitability for use in diverse and challenging environments.

In summary, microporous PTFE (or Polytetrafluoroethylene), distinguished by its hydrophobicity and intricate network of minuscule pores, serves as a versatile material for water filtration and gas diffusion applications. Its hydrophobic properties repel water, while its microporous structure allows for the easy passage of gases. Simultaneously, it effectively prevents liquid water from permeating the material due to the small size of its pores. The material's exceptional chemical resistance and wide temperature tolerance further enhance its suitability for various demanding conditions. This unique combination of features makes microporous PTFE an outstanding choice for applications requiring selective permeability to air and water.

Considering the present invention, depending on the embodiments, at least three ways of securing the membrane **70** to the sealed housing **25** of the control unit **20** may be found:

　　a. overhanging edge: the membrane **70** extends over the sealed housing's **25** surface, creating a protruding edge (see Figure 6a),
　　b. embedded edge: the membrane **70** is fixed in a way that it is embedded within the wall of the sealed housing **25**, resulting in a flush or recessed surface (see Figure 6c),
　　c. flush edge: the membrane **70** is fixed so that it is at the same level as the surface of the sealed housing **25**, producing a flush and even edge.

Considering the first securing way (a) listed here-above, preferably, each membrane **70** comprises an adhesive structure (ventilation patch) **700**. Each adhesive structure **700** is placed over its corresponding vent **50** from the inside **120** of the sealed housing **25**. This enables to decrease the risk that adhesive structure **700** is in direct contact with water. This involves creating an opening (vent **50**) inside the sealed housing **25**, then placing an adhesive ventilation patch **700** over the opening. The membrane **70** may positioned inside the housing **25** or outside the housing **25**.

The adhesive structure **700** is preferably adhered over the opening of each vent **50** onto flat plastic or metallic surfaces. The surface should be wiped dry before adhering. An initial period of a few hours is required for the adhesive to properly bond to the surface. Preferably, the adhesive structure **700** is not adhered onto the top-side of the opening of the vent **50**, as rain, water and dust will collect on the membrane **70**, which will weaken the adhesive seal and prevent correct ventilation.

In some embodiments, in which a higher level of protection is required, the adhesive structure **700** can be replaced by an embedded plastic or metal structure **701** secured inside the opening of the vent **50** (see Figure 6c). In some cases. Those structures are commonly called "screw-in" vent or a "push-fit" vent. They are available with different diaphragm types - and different air flow rates. The material of the membrane **70** is remains preferably Polytetrafluoroethylene.

In some particularly sensitive embodiments, a silicon membrane may be used.

Alternatively, the membrane **70** can be made of other material having hydrophobic and/or oleophobic properties, comprising (non-exhaustive list): saturated, monounsaturated and/or polyunsaturated polyamide, aliphatic polyamides, aromatic polyamides, polyurethanes, polyureaurethane, polyvinyl alcohol, polyacrylonitrile, polylactide, polycarbonate, polybenzimidazole, polyethylene oxide, polyethylene terephthalate, polybutylene terephthalate, polysulfone, polyvinyl chloride, cellulose, polyethylene, polypropylene, polyvinyl alcohol/silica, polyacrylonitrile/TiO2PETFE, polyetherimide, polyaniline, polyethylene naphthalate, styrene butadiene rubber, polystyrene, polyvinylidene fluoride, polyvinyl ethylene, polymethyl methacrylateMethyl methacrylate, or copolymers, derivative compounds, and blends and/or combinations thereof.

**Claims**

1. A wearable device (100) for measuring blood pressure of a subject comprising:

　　- a control unit (20) comprising a sealed housing (25), said sealed housing (25) comprising a pump (30);
　　- a cuff (10) configured to wrap around at least one portion of a limb of said subject, the cuff (10) comprising a bladder (15) configured to exert a pressure on the at least one portion of a limb of said subject, said bladder (15) being in fluidic connection with said pump (30) so as to be inflated;
　　- at least one vent (50) positioned on at least one wall (27) of said sealed housing (25), the at least one vent (50) being configured to connect an inside (120) of the sealed housing (25) with an outside environment (110) to allow air to be pumped in the sealed housing (25) by the pump (30) to inflate the bladder (15), *characterized in that* the at least one vent (50) comprises at least one waterproofing element (60, 70) configured to allow air to transit between the inside (120) of the sealed housing (25) and the outside environment (110) while preventing water from entering the sealed housing (25).

2. Device (100) according to claim 1, *wherein* the at least one vent (50) comprising the at least one waterproofing element (60, 70) is configured to allow air to transit with an air flow of at least 1 L/min.

3. Device (100) according to any of claims 1 or 2, *wherein* the at least one waterproofing element comprises a labyrinthine conduit (60) that connects the inside (120) of the sealed housing (25) with the outside environment (110) while preventing water from entering the sealed housing (25).

4. Device (100) according to any of claims 1 to 3, *wherein* said at least one vent (50) comprises an entry leading to the outside environment (110) and an exit leading to the inside (120) of the sealed housing (25) and wherein a pathway connects said entry to said exit, said pathway comprising at least one bend.

5.  Device (100) according to any of claims 1 to 4, *wherein* the at least one waterproofing element comprises at least one membrane (70).

6.  Device (100) according to claim 5, *wherein* the membrane (70) comprises pores with a diameter comprised between 0.02 $\mu$m and 0.5$\mu$m.

7.  Device (100) according to claim 6, *wherein* said pores have a density ranging from 70% to 90% and preferably of 82%.

8.  Device (100) according to any of claims 5 to 7, *wherein* the membrane (70) has a resistance of a minimum of 10 kPa, and preferably of a minimum of 30 kPa.

9.  Device (100) according to any of claims 5 to 8, *wherein* the membrane (70) is made of an hydrophobic material configured to repel water vapor molecules.

10. Device (100) according to any of claims 5 to 9, *wherein* the membrane (70) is made of polytétrafluoroéthylène (PTFE).

11. Device (100) according to any one of claims 5 to 10, *wherein* the membrane (70) comprises an adhesive structure (700) configured to be adhered on a portion of said at least one wall (27) of said sealed housing (25) wherein said at least one vent (50) is positioned, said portion surrounding said at least one vent (50).

**Fig. 1**

**Fig. 2**

**Fig. 3**

Fig. 4a

Fig. 4b

Fig. 4c

**Fig. 5a**

**Fig. 5b**

700

70

50

25

**Fig. 6a**

700

70

50

25

**Fig. 6b**

701

50 70

25

**Fig. 6c**

25

64

60

50

63

**Fig. 7a**

**Fig. 7b**

## EUROPEAN SEARCH REPORT

Application Number

EP 24 30 5365

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/290086 A1 (NISHIDA TOMOYUKI [JP]) 23 September 2021 (2021-09-23) * paragraphs [0005], [0010], [0011], [0012], [0065], [0074], [0078], [0107], [0123]; claims 1, 2, 5; figures 1,4,5,7 * | 1-11 | INV. A61B5/021 A61B5/00 |
| A | WO 2023/005847 A1 (HUAWEI TECH CO LTD [CN]) 2 February 2023 (2023-02-02) * claim 2 * | 2 | |
| A | CN 210 204 715 U (SHENZHEN KINGYIELD TECH CO LTD) 31 March 2020 (2020-03-31) * claims 1, 2 * | 3 | |
| A | EP 2 914 015 B1 (NITTO DENKO CORP [JP]) 17 July 2019 (2019-07-17) * paragraph [0132] * | 6-8,10 | |
| | | | TECHNICAL FIELDS SEARCHED (IPC) A61B H04B |

The present search report has been drawn up for all claims

| Place of search | Date of completion of the search | Examiner |
|---|---|---|
| The Hague | 7 May 2024 | Chacon Caldera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 30 5365

This annex lists the patent family members relating to the patent documents cited in the above-mentioned European search report.
The members are as contained in the European Patent Office EDP file on
The European Patent Office is in no way liable for these particulars which are merely given for the purpose of information.

07-05-2024

| Patent document cited in search report | | Publication date | Patent family member(s) | | Publication date |
|---|---|---|---|---|---|
| US 2021290086 | A1 | 23-09-2021 | CN | 113015483 A | 22-06-2021 |
| | | | DE | 112019005178 T5 | 01-07-2021 |
| | | | JP | 7175739 B2 | 21-11-2022 |
| | | | JP | 2020092886 A | 18-06-2020 |
| | | | US | 2021290086 A1 | 23-09-2021 |
| | | | WO | 2020121884 A1 | 18-06-2020 |
| WO 2023005847 | A1 | 02-02-2023 | CN | 115670411 A | 03-02-2023 |
| | | | EP | 4353148 A1 | 17-04-2024 |
| | | | WO | 2023005847 A1 | 02-02-2023 |
| CN 210204715 | U | 31-03-2020 | NONE | | |
| EP 2914015 | B1 | 17-07-2019 | CN | 104871554 A | 26-08-2015 |
| | | | CN | 105100328 A | 25-11-2015 |
| | | | EP | 2914015 A1 | 02-09-2015 |
| | | | HK | 1212846 A1 | 17-06-2016 |
| | | | JP | 5711412 B2 | 30-04-2015 |
| | | | JP | 5942004 B2 | 29-06-2016 |
| | | | JP | 2015065639 A | 09-04-2015 |
| | | | JP | 2015111945 A | 18-06-2015 |
| | | | KR | 20150063155 A | 08-06-2015 |
| | | | TW | 201510013 A | 16-03-2015 |
| | | | US | 2015304767 A1 | 22-10-2015 |
| | | | WO | 2015029302 A1 | 05-03-2015 |

EPO FORM P0459

For more details about this annex : see Official Journal of the European Patent Office, No. 12/82